# EUROPEAN PATENT SPECIFICATION

(11) **EP 0 648 222 B1**
(45) Date of publication and mention of the grant of the patent: **29.01.2003**
(21) Application number: 93918149.1
(22) Date of filing: 01.07.1993
(51) Int. Cl.: C07H 21/04, C12P 19/34

(54) **METHODS OF SINGLE NUCLEOTIDE PRIMER EXTENSION TO DETECT SPECIFIC ALLELES AND KITS THEREFOR**
VERFAHREN ZUR EINFACHEN NUKLEOTID-PRIMER-VERLÄNGERUNG ZUM NACHWEIS SPEZIFISCHER ALLELE UND DAFÜR GEEIGNETER KIT
PROCEDE D'ALLONGEMENT D'AMORCE DE NUCLEOTIDE SIMPLE BRIN DESTINE A DETECTER DES ALLELES SPECIFIQUES ET TROUSSES PERMETTANT D'APPLIQUER LEDIT PROCEDE

(30) Priority: 02.07.1992 IL 10238292; 27.07.1992 US 919872
(43) Date of publication of application: 19.04.1995
(73) Proprietor: SAVYON DIAGNOSTICS LTD., 77610 Ashdod (IL)
(72) Inventor: EYAL, Nurit, 76 345 Rehovot (IL)
(74) Representative: Müller, Frank Peter, Dipl.-Ing.
(86) International application number: US9306364
(87) International publication number: WO94001447

(56) References cited:
- EP-A- 0 412 883
- WO-A-90/09455
- WO-A-91/13075
- WO-A-92/15712
- WO-A-92/16657
- US-A- 5 137 806
- Nucleic Acids Research, Volume 18, Number 12, issued 25 June 1990, B.P. SOKOLOV, "Primer Extension Technique for the Detection of Single Nucleotide in Genomic DNA", page 3671, see entire document.
- Proc. Natl. Acad. Sci. (USA), Volume 28, issued February 1991, M.N. KUPPUSWAMY et al., "Single Nucleotide Primer Extension to Detect Genetic Diseases: Experimental Application to Hemophilia B (Factor IX) and Cystic Fibrosis Genes", pages 1143-1147, see entire document.
- Science, Volume 238, issued 1987, J.M. PROBER et al., "A System for Rapid DNA Sequencing with Fluorescent Chain-Terminating Dideoxynucleotides", pages 336-341, see entire document.
- PCR Methods and Applications, Volume 1, issued 1992, J. SINGER-SAM et al., "A Sensitive, Quantitative Assay for Measurement of Allele-specific Transcripts Differing by a Single Nucleotide", pages 160-162, see entire document.
- Genet. Anal. Tech., Volume 7, issued 1990, E. HORNES et al., "Magnetic DNA Hybridization Properties of Oligonucleotide Probes Attached to Super Paramagnetic Beads and their Use in the Isolation of Poly(A) mRNA from Eukaryotic Cells", pages 145-150, see entire document.

## Description

### FIELD AND BACKGROUND OF THE INVENTION

The present invention relates to the identification of specific nucleotide sequences and to the detection of mutations at particular sites within nucleotide sequences.

More particularly, the present invention concerns a method and kit for the detection of the presence of a certain sequence in a sample of genetic material. The method and kit of the present invention are highly sensitive to small alterations in the sequence and thus are useful in the detection of point mutations, i.e., single base-pair alterations in a DNA sequence.

The present method and kit are also useful for identifying the presence of foreign genetic material in a sample of genetic sequence, for example, for detecting the presence of specific bacterial or viral nucleotide sequences in plant and animal DNA.

In recent years with the development of methods such as polymerase chain reaction (PCR) and various automated DNA sequencing techniques, an extremely large number of human genes have been isolated, identified and fully sequenced. One of the consequences of such developments has been the elucidation of the genetic basis of many diseases such as, for example, Cystic Fibrosis, Hemophilia, Lesch-Nyhan syndrome, β-thalassemia. Sickle Cell Anemia, Phenylketonuria, Tay-Sachs, Gaucher and many others. A large number of genetic diseases have been shown to be caused by point mutations in the gene or by deletion or insertion of a known number of nucleotides. As a consequence of such point mutations or small sequence alterations the protein encoded by such genes is not produced, prematurely truncated or is produced in a modified form which affects its function. Furthermore, many cancers have been shown to be associated with point mutations in certain genes.

In view of the aforementioned development, it is now possible to obtain genetic material from an individual, amplify a certain gene region using PCR technology, and then sequence this region using commercially available sequencing techniques. The sequenced gene region may then be compared with the known normal sequence to determine whether the individual has a mutation at any particular site in this region. In this way, it is possible to determine whether an individual has a certain disease or whether an individual is a "carrier", i.e., is heterozygous for the mutation of the site tested. When the sequencing is performed on fetal cells, it becomes possible to determine the chances that the fetus will bear a certain inherited disease. This may allow the treatment of the disease shortly after birth using special diets or medicines or using genetic therapy, or, if treatment is not possible, offers the option of terminating the pregnancy.

Such techniques have also become important in a number of other applications including in forensic medicine where typically only minute samples are available, in question of paternity, and in the analysis of a sample for the presence of the DNA of a specific pathogen, for example, DNA of viral origin such as HIV.

The most basic method for detection of point mutation is sequencing, the most widely used sequencing method being based on the dideoxynucleotide chain termination procedure. The technique involves the incorporation of dideoxynucleotides with the aid of a DNA polymerase at the 3' end of an elongating DNA chain. Once the dideoxynucleotide has been incorporated, further elongation of the chain is blocked. See, Sanger F. (1981), Science 214, 1205-1210.

Recently automated DNA sequencing techniques have been developed which provide for more rapid and safer DNA sequencing. One such approach utilizes a set of four chain-terminating fluorescently-labelled dideoxynucleotides. See Chehab, F.F., et al. (1989), Proc. Natl. Acad. Sci. (USA) 86, 9178-9182; Prober, J.M., et al. (1987), Science 238, 336-341; Smith, L.M., et al. (1980), Nature 321, 674-678). In this method succinyl fluorescein dyes are used. Each dideoxynucleotide receives a different dye of different absorption and emission characteristics. Thus, DNA molecules labelled with each of the different dideoxynucleotides may be distinguished from one another. Using these dideoxynucleotides, it is possible to sequence a DNA segment by carrying out a single reaction in which all four of the differently labelled dideoxynucleotides are added together into a single reaction mixture and the resulting labelled oligonucleotide fragments may then be resolved by polyacrylamide gel electrophoresis in a single sequencing lane on the gel. The gel is then scanned by a fluorimeter capable of distinguishing the different fluorescent labels. The sequence of the different labels along the lane is then translated into the sequence of the tested DNA segment.

Other methods which have been used to determine the presence of point mutations in known DNA sequences include ligase chain reaction (LCR), a modification of the PCR method in which the ligated pair of oligomers serve as template for the subsequent ligation and thus increase the concentration of ligated product in an exponential manner. Another common technique involves Allele Specific Oligonucleotide (ASO) in which hybridization is carried out under stringent conditions such that only the labeled probe with complete homology to the template remains hybridized and thus determines the sequence of the template. The very specific reaction conditions leave little margin for error and, unless carried out by highly skilled personnel.

More recently, a novel method for the detection of point mutations has been disclosed which is based on a single nucleotide primer extension. See, Sokolov, 1989, Nucleic Acids Research 18(12), 3671; Kuppuswamy, M.H., et al. (1.991), Proc. Natl. Acad. Sci. (USA) 88, 1143-1147; Singer-Sam, J., et al., (1992), in PCR Methods and Applications. Cold Spring Harbor Laboratory Press, Cold Spring Harbor, New York, U.S.A., pp. 160-163. In this method, the DNA containing the putative mutation site is tested or first amplified by the use of PCR. Several reaction mixtures are then prepared and for each amplified fragment. Each reaction mixture contains a primer whose sequence is complementary to the genomic sequence adjacent to the nucleotide to be determined. The mixture further includes a radioactively labelled nucleotide corresponding to the normal coding sequence at the tested site or to a suspected mutant sequence at the site, and a DNA polymerase catalyzing the incorporation of the radio-labelled nucleotide into the primer, or, equivalently, the extension of the primer by the radio-labelled nucleotide. The primers are then separated from the template and the occurrence of radiative labelling on the primers is determined. On this basis the subject may be identified as being normal (non-mutated, wild type) or a heterozygous or homozygous for the tested point mutation.

WO 90/09455 discloses a method for detecting a nucleotide or base change, wherein a 5'labeled specific primer is hybridized to a target DNA. The label, which marks the primer, is called the detector molecule D. According to said patent application typically detector molecules are represented by a radioactive label selected from the group consisting of ³H, ¹²⁵I, ¹³¹I, ¹⁴C, ³⁵S and ³²P. In a specific embodiment of said patent application the 3'end of the primer carries a molecule which is used for separating the labeled primer and which is called the capture molecule C. With respect to the possible kind of capture molecules WO 90/09455 states that a capture molecule is a molecule which can covalently be attached to a nucleotide or nucleotide sequence and which will subsequently bind to a specific affinity molecule, for example biotin, antibody, antigen.

WO 91/13075 discloses the detection of variable nucleotides based on primer extension and the incorporation of detectable nucleoside triphosphates. Accordingly a 5'biotinylated primer is used during the amplification of the DNA by polymerase chain reaction, thereby incorporating biotin at the 5'end of the primers used for DNA amplification. Thus, according to said prior art document a capture molecule or a separation molecule, respectively, is incorporated already into DNA before a process for determining whether or not one of one or more given nucleotide bases are present at a specific position in a target nucleic acid contained in a sample can take place. Moreover, for detecting whether a variable nucleotide has been incorporated into the strand complementary to the strand of the target nucleic acid said patent application uses the incorporation of digoxigenin-11-dUTP into the strand used for the primer extension reaction.

EP-A 0 412 883 discloses a microsequencing process for determining the existence of a point mutation downstream of the 3'end of a primer, wherein a modified base is used, e.g. ddNTPs.

While these aforementioned methods present improvements in point mutation detection, a more accurate and more advanced method is presented herein which better addresses the detection of point-mutated and foreign genetic material.

It is an object of the present invention to provide an improved method allowing the identification of mutations at specific sites within a certain nucleotide sequence, said method being fast and simple and providing reliable results.

These objects are achieved by a method for determining whether or not one of one or more given nucleotide bases is present at a specific position in a target nucleic acid contained in a sample, said method comprising the steps of
(a) if the target nucleic acid in said sample is double-stranded, treating said sample to render a region spanning said specific position single-stranded; or, if the target nucleic acid in said sample is single stranded, proceeding directly to step (b); and
(b) forming a hybrid between said target nucleic acid in said sample and a fluorescently labeled oligonucleotide primer, said primer hybridizing to a region on said target that is directly downstream (3') from said specific position, such that a nucleotide base added to the 3'end of said primer would be complementary to said specific position; and
(c) contacting said hybrid with one or more primer extension units, said primer extension unit including an extension moiety and a separation moiety, which is selected from the group consisting of antibodies, antigens, haptens, lectins and carbohydrates, in the presence of a template-dependent extension enzyme under conditions such that if said extension moiety of one of said primer extension units is complementary to said specific position, said primer extension unit will become covalently attached to the 3'end of said primer; and
(d) contacting said primer with a solid support having an affinity for the separation moiety of the primer extension unit, under conditions that promote the attachment of said separation moiety to said suport; and
(e) determining whether said labeled primers have substantially become attached to said solid support, such substantial attachment indicating that the nucleotide base present at said specific position is a base complementary to one of the one or more extension moieties used, and the substantial absence of such attachment indicating that said nucleotide base is not a base that is complementary to one of the one or more extension moieties used.

Preferred embodiments of the method according to the invention are the subject of the dependant claims.

### SUMMARY OF THE INVENTION

According to the present invention, there is provided a method useful in determining the identity of a nucleótide base at a specific position in a nucleic acid of interest, said method using an aqueous carrier and at least one primer extension unit, the primer extension unit including an extension moiety and a separation moiety, the extension moiety capable of specifically terminating a nucleic acid template-dependent, primer extension reaction, in a manner which is strictly dependent on the identity of the unpaired nucleotide base of the template immediately adjacent to, and downstream of, the 3' end of the primer, the separation moiety permitting the affinity separation of the primer extension unit from unincorporated reagent and nucleic acid.

According to features of the method according to the invention, the removal of the non-extended marked primer includes: (i) attaching the separation moiety of the primer extension unit to a solid support; and (ii) removing the marked primer not connected to the solid support.

According to further features in preferred embodiments of the invention described below and in the dependent claims, the separation moiety is capable of attaching to a solid support. An example of such a separation moiety is biotin which is capable of attaching to a solid support coated with avidin, streptavidin or antibiotin.

According to still further features in the described preferred embodiments, the extension moiety is a deoxynucleotide, such as dATP, dCTP, dGTP, dTTP and dUTP, or a dideoxynucleotide, such as ddATP, ddCTP, ddGTP, ddTTP and ddUTP.

A specific application of the method of the present invention is in the identification of point mutations at specific sites in a gene. The primer features a sequence which is complementary to the normal gene at a site downstream of, and immediately flanking, the putative mutation site. The primer preferably also features a suitable marker as will be discussed in more detail below.

Suitable extension moieties of primer extension units include those in which the hydroxyl group normally found attached to the 3' carbon is replaced with a different moiety such that once the primer extension unit is incorporated into an oligonucleotide primer, no other nucleotide may be bound to this modified nucleotide. Examples of such extension moieties include moieties wherein the 3' OH groups has been replaced by H, SH and the like, including, but not limited to, various other substituent groups. Examples of extension moieties of primer extension units are deoxynucleotides or dideoxynucleotides or their analogs. The extension moiety is attached to a suitable fluorescent label.

The primers may be of any suitable length. Time and expense considerations tend to shift preference toward shorter primers which are still sufficiently long to ensure high sequence specificity while at the same time ensuring rapid, easy and accurate preparation. The primer can be substantially or precisely complementary to the complementary portion of the nucleic sequence being examined. The sample of genetic material being tested by the above method may be in the form of RNA or DNA.

### BRIEF DESCRIPTION OF THE DRAWINGS

The invention is herein described, by way of example only, with reference to the accompanying drawings, wherein:
FIG. 1 is a schematic outline of features of a basic method for detecting point mutation in a known gene by means of nucleotide extension;
FIG. 2 is a schematic outline of features of a preferred embodiment of a method according to the present invention for detecting point mutations in a known gene;
FIG. 3 is a schematic depiction of the operation of the basic method of Figure 1 when applied to detecting a 3 bp deletion mutation in the CFTR gene;
FIG. 4 is a schematic depiction of the operation of a preferred 5 embodiment of Figure 2 when applied to detecting a 3 bp deletion mutation in the CFTR gene;
FIG. 5 is a schematic depiction of the operation of a preferred embodiment for the simultaneous detection of three mutations in two vessels.

### DESCRIPTION OF THE PREFERRED EMBODIMENTS

The present invention is of a novel method for of determining the identity of a nucleotide base at a specific position in a nucleic acid of interest, and of a reagent for use in implementing the method.

The principles and operation of a method according to the present invention may be better understood with reference to the drawings and the accompanying description.

The present invention will be described in more detail with emphasis on a method for identifying point mutations in genes, which mutations are associated with genetic disorders. While this application of the method of the invention is presently preferred, this is by no means the only application of the invention as will no doubt be appreciated by those skilled in the art. For example, the method has various other applications including, but not limited to, the detection of specific genetic sequences in a sample such as those associated with certain genetic diseases and pathogenic microorganisms, for example bacteria and viruses in testing of paternity and in forensic medicine, cancers and plant polymorphisms.

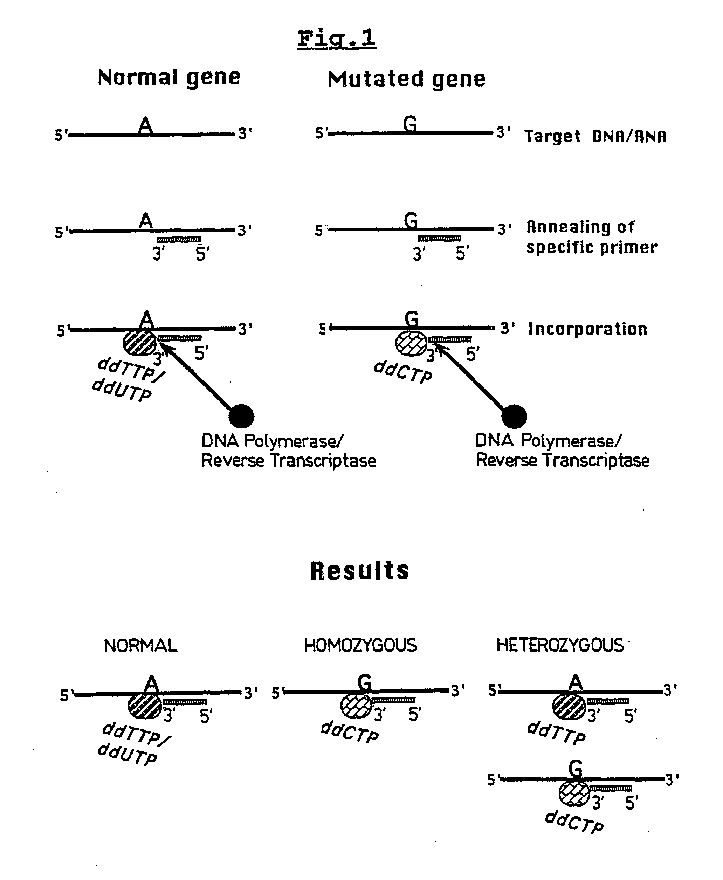

In order to better understand the present invention, reference is made to Figure 1 which is a schematic depiction of a basic method. A description of a related method is given in PCT/US92/01905 (WO 92/15712).

In order to letter understand the preferred methods according to the present invention it is useful to first describe the basic method. To test for the occurrence of an A → G point mutation of a certain position in a known gene, a sample of DNA is obtained and a specific primer having a sequence which is complementary to the sequence of the region downstream of and immediately flanking the 3' end of the suspected mutation site, is annealed to the sample. Labelled dideoxynucleotides are then added together with a DNA polymerase. Following the incorporation reaction the primers are tested for incorporation of a terminator. In the case of a normal individual, only dideoxythymidine (ddTTP) will be incorporated. Where the individual is homozygous for the A → G mutation at that site, all primers will incorporate dideoxycytosine (ddCTP). If the individual is heterozygous, i.e., one of its alleles is normal and the other is mutated, some primers will incorporate dideoxythymidine and others dideoxycytosine.

In its simplest form, the basic method for identifying point mutation may be summarized as follows:
(i) A sample of genetic material in the form to be analyzed is obtained in an aqueous carrier and annealed to a specific oligonucleotide primer having a sequence complementary to the template sequence downstream of, immediately flanking, the site of interest, e.g., the mutation site;
(ii) A set of one to four labelled chain elongation terminator nucleotides, for example, terminator nucleotide such as the dideoxynucleotides ddATP, ddCTP, ddGTP, ddTTP and ddUTP, or analogs thereof, are added to the mixture of (i);
(iii) A DNA polymerase is then added in an appropriate buffer, and an incorporation, or extension, reaction of the terminator nucleotide is thereby initiated;
(iv) After incorporation, or extension, a series of appropriate washes are carried out to remove the non-extended labelled terminator nucleotide, which removal can alternatively be accomplished using gel separation techniques; and
(v) Labelling of the nucleotide primer is then determined by suitable analytical means, the labelling indicating the incorporation of a terminator nucleotide to the primer, and therefore provides an indication of the identity of the specific nucleotide base-pair present at the mutation site.

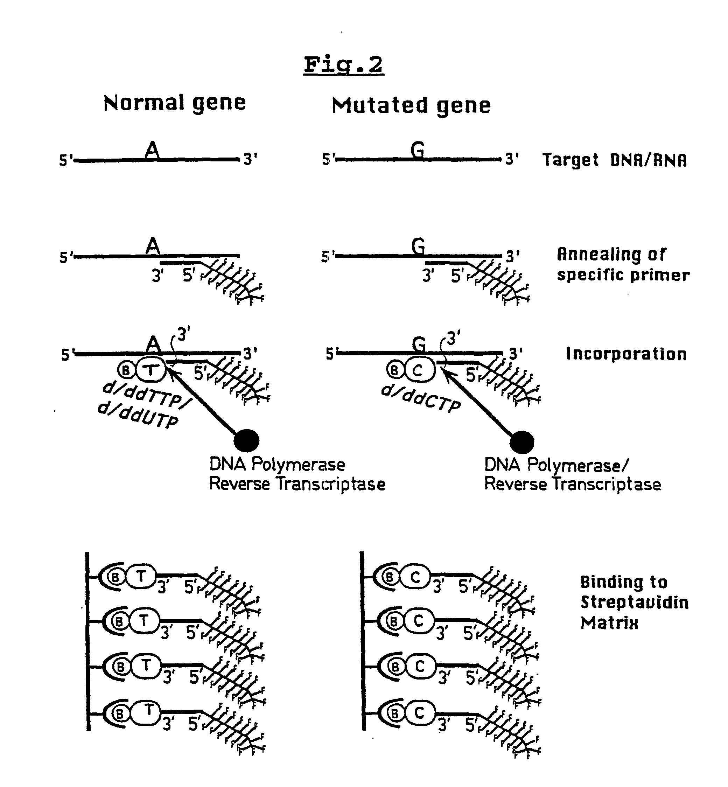

In a preferred embodiment of the method of the present invention, depicted schematically in Figure 2, the primer used is labeled with a suitable marker while the primer extension unit includes a suitable separation moiety as stated in claim 1, such as a suitable hapten, which could be, for example, dinitrophenol (DNP), digoxigenin (DIG), sulfur species and the like, preferably, biotin, which facilitates the affinity separation of the extended primer.

More specifically, under this preferred embodiment, the identity of a nucleotide base at a specific position in a nucleic acid of interest can be determined by carrying out a process, somewhat similar to that described above but using a marked primer and a biotinylated primer extension unit. Thus, if the nucleic acid is double-stranded, a sample containing the nucleic acid of interest is first treated to obtain unpaired nucleotide bases spanning the specific position. If the nucleic acid of interest is single-stranded, this step can be skipped.

The unpaired nucleotide bases spanning the specific position are then contacted, under hybridizing conditions, with the marked oligonucleotide primer which is capable of hybridizing with a stretch of nucleotide bases present in the nucleic acid of interest adjacent to, and downstream of, the nucleotide base to be identified. The result is the formation of a duplex between the primer and the nucleic acid of interest such that the nucleotide base to be identified is the first unpaired base in the template immediately downstream of the 3' end of the primer in the duplex.

A special reagent useful in determining the identity of a nucleotide base at a specific position in a nucleic acid of interest includes an aqueous carrier and one or more primer extension units. The primer extension unit includes an extension moiety and a separation moiety described above which are connected together. The extension moiety is capable of specifically terminating a nucleic acid template-dependent, primer extension reaction in a manner which is strictly dependent on the identity of the unpaired nucleotide base of the template immediately adjacent to, and downstream of, the 3' end of the primer. The separation moiety permits the affinity separation of the primer extension unit from unincorporated reagent and nucleic acid.

The duplex formed above is contacted with a special reagent, such as that described above, under conditions permitting the base pairing of the complementary extension moiety of the primer extension unit present in the reagent with the nucleotide base to be identified and the occurrence of a template-dependent, primer extension reaction to incorporate the extension moiety of the primer extension unit at the 3' end of the primer, resulting in the extension of the primer by a single unit.

Once the extension of the marked primer by the primer extension unit has been effected, any non-extended marked primers are removed by any suitable technique and any suitable technique is used to determine the identity of the extended primer.

The primer may be marked with any suitable fluorescent marker.

The separation moiety of the primer extension unit is capable of attaching to a solid support. The attachment to the solid support may be accomplished through the coating of the solid support with avidin, streptavidin, antibiotin or certain antibodies.

Preferably, the separation moiety of the primer extension unit includes biotin which permits affinity separation of the primer attached to the extension moiety of the primer extension unit from the unincorporated reagent and from the nucleic acid of interest through the binding of the biotin to streptavidin, avidin or antibiotin attached to a solid support.

Any suitable fluorescently labeled extension moiety of the primer extension unit may be used. Preferably, the extension moiety is deoxynucleotide, such as dATP, dCTP, dGTP, dTTP and dUTP , most preferably a dideoxynucleotide, such as ddATP, ddCTP, ddGTP, ddTTP and ddUTP.

The removal of the non-extended marked primer may be accomplished in various ways. Before the removal can be effected it is normally necessary to separate the primer from the nucleic acid of interest. This is typically accomplished by use of appropriate denaturing conditions.

Preferably, the removal of the non-extended marked primer is effected by first attaching the separation moiety of the primer extension unit to a solid support and then removing, as by washing, any marked primer not connected to the solid support.

The identity of the extended primer can be determined in any suitable way, depending on the mature of the marker used to mark the primer and other factors. Preferably, the determination of the identity of the extended marked primer, if any, is carried out following its attachment to the solid support via the separation moiety of the primer extension unit. Thus, if the primer extension unit was able to extend the primer, the marked primer will be detected as being attached to the solid support. On the other hand, if the primer extension unit was not able to extend the primer, only the primer extension units, and no primers, will be attached to the solid support, leading to the failure to detect the marked primers.

Methods according to the present invention enjoy a number of advantages relative to the basic method described above. First, the marking of the primer, rather than of the terminator, can be done in such a way as to incorporate a large and unmistakable marker, e.g., a relatively long chain of fluorescent moieties, which can be easily picked up by simple fluorometers. It is estimated that the marked primer may be up to 30 times easier to detect than the marked terminators used in the basic method.

Second, the marked primers used are much easier to produce than the marked fluorescent terminators of the basic method.

Third, marked fluorescent terminators are highly polymerase-dependent. See, for example, Lee, L.G. et al., Nucleic Acids Research, 1992, 20(10) 2471-2483, 2472, which states that a disadvantage of dye-labeled terminators is that they must be tailored to a specific DNA polymerase.

Fourth, fluorescently marked primers used are more stable than the corresponding marked terminators of the basic method. An added complication with the marked terminators of the basic method is that the different terminators which are marked in the same way display different degrees of stability. See, for example, Voss, H., Methods in Molecular and Cellular Biology. These complications significantly limit the applicability of the basic method in commercial tests.

Finally, methods the present invention have the advantage over the basic method in that they can be used to simultaneously investigate a number of different mutations involving the same base pair. By marking each of several different primers with a different marker, e.g., green, red, and blue fluorescence, it is possible to analyze three separate mutations simultaneously, as is described in more detail in Example 3.

The genetic material to be analyzed may, in principle, be any RNA or DNA obtained from the tissues or body fluids of humans, animals or plants or obtained from cultures of microorganisms or human, animal or plant cells or nucleic acid synthesized by polymerase. The genetic material may alternatively be obtained from non-living sources suspected of containing matter from living organism sources, as may be the case when applying the method in forensic medicine for detecting and identifying specific nucleotide sequences present in or on samples of clothing, furniture, weapons and other items found at the scene of a crime. In this instance, the genetic material obtained is usually in the form of DNA, since any RNA in such samples would normally have been degraded by ribonucleases.

The specific application of the inventive method for the detection and identification of mutations and/or polymorphisms in genes having a known sequence is presently a preferred embodiment. In this application the method may be used as a diagnostic assay to determine the specific mutation present in an individual suffering from, or showing symptoms of, a disease known to be caused by one or more point mutations in a specific gene. The method may also be applied for screening healthy individuals to determine whether they are carriers, i.e., heterozygous for point mutations linked to known diseases. This is the case, for example, in the well elucidated Tay-Sachs disease in which diseased individuals have mutations in both alleles encoding the hexoaminidase A gene, and carriers of the disease have one or more mutations in one allele only. Furthermore, the method may also be applied for screening embryos by analyzing samples of amniotic fluid to determine whether the embryos have mutations in one or two or none of the alleles encoding a gene known to be involved in a specific disease.

The sample of nucleic acids can be drawn from any source and may be natural or synthetic. The sample of nucleic acids may be made up of deoxyribonucleic acids, ribonucleic acids, or copolymers of deoxyribonucleic acid and ribonucleic acid. The nucleic acid of interest can be synthesized enzymatically in vitro, or synthesized non-enzymatically. The sample containing the nucleic acid or acids of interest can also comprise extragenomic DNA from an organism, RNA transcripts thereof, or cDNA prepared from RNA transcripts thereof. Also, the nucleic acid or acids of interest can be synthesized by the polymerase chain reaction.

The oligonucleotide primer may be any suitable species, preferably an oligodeoxyribonucleotide, an oligoribonucleotide, or a copolymer of deoxyribonucleotides and ribonucleotides. The oligonucleotide primer can be either natural or synthetic. The oligonucleotide primer can be synthesized enzymatically in vivo, enzymatically in vitro, or non-enzymatically in vitro. The oligonucleotide primer is labeled with a fluorescently detectable marker. The marker can be different from any detectable marker present in the reagent or attached to the nucleic acid of interest. In addition, the oligonucleotide primer must be capable of hybridizing or annealing with nucleotides present in the nucleic acid of interest, immediately adjacent to, and downstream of, the nucleotide base to be identified. One way to accomplish the desired hybridization is to have the template-dependent primer be substantially complementary or fully complementary to the known base sequence immediately adjacent the base to be identified.

The oligonucleotide primers can be any length or sequence, can be DNA or RNA, or any modification thereof. It is necessary, however, that the length of the primer be chosen to optimize the specificity of the hybridization to the target sequences of interest.

The primer can be separated from the nucleic acid of interest after the extension reaction by using appropriate denaturing conditions, which may include heat, alkali, formamide, urea, glyoxal, enzymes, and combinations thereof.

Different versions of the method for determining the identity of a nucleotide base at a specific position in a nucleic acid of interest and the method for determining the presence or absence of a particular nucleotide sequence in a sample of nucleic acids are possible. In one version, the template is deoxyribonucleic acid, the primer is an oligodeoxyribonucleotide, oligoribonucleotide, or a copolymer of deoxyribonucleotides and ribonucleotides, and the template-dependent enzyme is a DNA polymerase, yielding a DNA product.

In a second version, the template is a ribonucleic acid, the primer is an oligodeoxyribonucleotide, oligoribonucleotide, or a copolymer of deoxyribonucleotides and ribonucleotides, and the template-dependent enzyme is a reverse transcriptase, yielding a DNA product.

In a third version, the template is a deoxyribonucleic acid, the primer is an oligoribonucleotide, and the enzyme is an RNA polymerase, yielding an RNA product.

In a fourth version, the template is a ribonucleic acid, the primer is an oligoribonucleotide, and the template-dependent enzyme is an RNA replicase, yielding an RNA product.

The nucleic acid of interest may be non-natural nucleotide analogs such as deoxyinoside or 7-deaza-2'-deoxyguanosine. These analogs destabilize DNA duplexes and could allow a primer annealing and extension reaction to occur in a double-stranded sample without completely separating the strands.

A method according to the present invention can be used to determine the identity of a nucleotide base at different alleles of a specific position in a nucleic acid of interest. The procedure is as described above but using at least two separate vessels. The reagent used in each vessel contains a primer extension unit having a different extension moiety.

A method according to the present invention can also be used to type a sample containing nucleic acids. Such a process includes identifying the nucleotide base or bases at each of one or more specific positions, each such nucleotide base being identified using the method as described above, and each such specific position being determined using a different primer.

A method according to the present invention can be further used to identify different alleles in a sample containing nucleic acids. Such a process includes identifying the nucleotide base or bases present at each of one or more specific positions, each of such nucleotide bases being identified by the method described above.

Another application of a method according to the present invention is in the determination of the genotype of an organism at one or more particular genetic loci. Such a process calls for obtaining from the organism a sample containing genomic DNA. The nucleotide base or bases present at each of one or more specific positions in nucleic acids of interest is identified by the process described above. In this way, different alleles are identified and, in turn, the genotype of the organism is determined at one or more particular genetic loci.

The subject invention also provides a method of typing a sample of nucleic acids which comprises identifying the base or bases present at each of one or more specific positions, each such nucleotide base being identified using one of the methods for determining the identity of a nucleotide base at a specific position in a nucleic acid of interest as outlined above. Each specific position in the nucleic acid of interest is determined using a different primer. The identity of each nucleotide base or bases at each position can be determined individually or the identities of the nucleotide bases at different positions can be determined simultaneously.

The subject invention also provides another method of typing a sample of nucleic acids which comprises determining the presence of absence of one or more particular nucleotides sequences, the presence of absence of each such nucleotide sequence being determined using one of the methods for determining the presence or absence of a particular nucleotide sequence in a sample of nucleic acids as outlined above.

The subject invention also provides an additional method of typing a sample containing nucleic acids. First, the presence or absence of one or more particular nucleotide sequences is determined; the presence or absence of each such nucleotide sequence is determined using one of the methods for determining the presence or absence of a particular nucleotide sequence in a sample of nucleic acids as outline above. Second, the nucleotide base or bases present at each of one or more specific positions is identified; each such base is identified using one of the methods for determining the identity of a nucleotide base at a specific position in a nucleic acid of interest as outlined above.

The subject invention further provides a method for identifying different alleles in a sample containing nucleic acids which comprises identifying the base or bases present at each of one or more specific positions. The identity of each nucleotide base is determined by the method for determining the identity of a nucleotide base at a specific position in a nucleic acid of interest as outlined above.

One or more primer extension units as described above, in combination with one or more appropriate marked oligonucleotide primers, and a DNA polymerase, and an appropriate salt and cofactor mixture, can be used under appropriate hybridization conditions as a kit for diagnosing or typing nucleic acids. The kit further includes an appropriate solid support and suitable buffers, such as binding solution and wash solutions.

The conditions for the occurrence of the template-dependent, primer extension reaction can be created, in part, by the presence of a suitable template-dependent enzyme. Some of the suitable template-dependent enzymes are DNA polymerases.

Table I lists a sampling of the various diseases which are known to result from the presence of one or more mutations in a gene encoding a specific protein or enzyme. Most of these diseases are recessive diseases, i.e., the diseased individual has both alleles carrying a mutation, the mutation resulting in the protein being absent (gene not expressed), in an inactive state (having an altered amino acid sequence), or being present in less than the required amounts (significantly reduced gene expression).

**TABLE I**

| DISEASE | GENE |
|---|---|
| Hemophilia A | factor VIII |
| Hemophilia B | factor IX |
| Lesch-Nyhan syndrome | HPRT |
| Ornithine transcarbamylase | OTC |
| Hereditary Amyloidosis (HA) | transthyretin (TTR) |
| Gaucher | glucocerebrosidase |
| Cystic fibrosis | CFTR |
| Osteogenesis imperfecta | collagen (I, II), procollagen |
| Hemoglobinopathies (e.g., β-thalassemia, Sickle cell anemia) | hemoglobin |
| Acute intermittent porphyria (AIP) | pophobilinogen deaminase |
| Phenylketonuria | phenylalamine hydroxylase |
| Tay Sachs | hexosaminidase A (HEXA) |
| Familial hypercholesterolemia (FH) | LDL receptor |
| Neurofibromatosis | NF1 |

The ongoing research to determine the genetic basis for diseases and the advent of technologies such as the polymerase chain reaction (PCR) has resulted in the discovery and complete sequencing of more and more genes encoding structural protein or enzyme products, a mutation in which would lead to either no expression of the gene product or expression of a product which is qualitatively or quantitatively impaired and thereby resulting in a disease. There is thus an ever expanding field of application of the above method of the invention.

The method of the invention, besides having use in diagnosis of specific disease-linked mutations in known gene regions, may also be of use in testing for the presence of a specific sequence associate with blood typing, tissue classification - HLA-typing, sex determination or possible susceptibility of an individual to certain diseases. Tissue classifications, for example, may be determined by identifying polymorphisms being specific for a particular individual. Screening these known HLA gene sequences by the present method may also be used as a diagnostic tool to determine whether the individuals in question are susceptible to certain diseases, e.g., various specific autoimmune diseases which are correlated with the specific HLA genes carried by the individual.

As noted above, the method of the invention may also be applied in the field of forensic medicine in which polymorphisms in specific genes, e.g., the β-globin gene cluster and the various known repeat sequences, can be determined in, for example, blood or semen samples obtained at the scene of a crime and the results used to indicate whether or not a particular suspect was involved in the crime. Similarly, the aforesaid determination may also be used to determine whether a certain male individual is the father in cases of disputed paternity.

There is evidence that certain cancers may be the result of specific point mutation in the sequence of certain genes and, accordingly, the present methods may be used as an early diagnostic tool to screen the general population or those individuals considered most likely to develop such cancers.

Another application of the present methods, as noted above, is the detection of microorganisms in a sample on the basis of the presence of specific sequences in the sample. For example, an individual suspected of being infected by a microorganism, such as a bacteria or virus, can be tested by using a specific oligonucleotide which anneals only with a specific bacterial or viral DNA sequence and not with sequences present in the individual. One example of such an application is in the screening of individuals for the presence of the AIDS virus. Moreover, by application of the present method the specific strain of virus, e.g., HIS-I, HIV-II, or HIV-III, may also be determined in a sample. Similarly, different species or strains of bacteria in a sample may be distinguished one from the other, e.g., the presence of Shigella vs. Salmonella bacteria which are difficult to distinguish from one another by standard techniques.

Gene regions corresponding to all of those set forth in Table I above and many others, may be analyzed for the presence of one or more point mutations at any number of sites within the gene region, or the existence of polymorphisms for any specific allele, or whether the individual being tested is homozygous for a specific base pair mutation, heterozygous therefor (i.e. carrier) or whether the individual is normal for this specific base pair (i.e. carrying two normal genes).

The present method can be a very effective alternative for the traditional mutation methods which use radioactive material, different hybridization or PCR conditions for every mutation, specific gels or an expensive automated sequencer. The present method enables a large-scale diagnostic procedure for multi-mutation detection with the possibility of screening many different samples in a short period of time. Furthermore, the present method provides a means for population screening of the multi-mutations in a wide range of inherited diseases and genetic disorders such as genetic cancers and the like, and can also be easily adapted for screening polymorphisms such as those in HLA genes, or detecting for the presence of pathogenic RNA or DNA, or the differentiation among different strains of bacteria or viruses.

The invention will now be further illustrated by the following examples:

### EXAMPLES

### a) Detecting and identifying mutation in cystic fibrosis gene region

The cystic fibrosis (CF) gene has been cloned and the cDNA therefor has been completely sequenced. See, for example, Rommens, J.M., et al. (1989), Science 245, 1059-1065; Riordan, J.R., et al. (1989), Science 245, 1066-1073; Kerem, B., et al. (1989), Science 245, 1073-1080. The CF gene is more than 250 kb in length and encodes a transcript of about 6.5 kb in length, the transcript encoding sequences of the gene being divided amongst 27 exons. The protein encoded by the gene, i.e., translated from the aforesaid transcript, is 1480 amino units in length having a molecular weight of about 168 Kd. Due to its putative role in the regulation of ion transport across the membrane, the CF protein and hence the CF gene has also been renamed the cystic fibrosis transmembrane conductance regulator (CFTR protein and CFTR gene).

Since the elucidation of the complete cDNA sequence many patients suffering from cystic fibrosis have been tested for the presence of mutation within the CFTR gene in an attempt to understand the molecular basis for the disease. From these studies it has been observed that a deletion of three base pairs within exon No. 10, which results in the loss of a single codon, No. 508, encoding a phenylalanine residue is the most frequent mutation among cystic fibrosis patients and causes cystic fibrosis with pancreatic insufficiency. To date, more than 300 additional mutations, each of low frequency in the studied populations, have been reported.

Existence of such a large number of different mutations of low frequency has made it difficult to detect and identify mutations in cystic fibrosis patients. All of the aforementioned mutations were identified following the laborious procedure of isolating and sequencing the CFTR genes of cystic fibrosis patients. Accordingly, to positively diagnose a suspected cystic fibrosis patient and to identify the exact mutation in the CFTR gene causing this condition has up to now been an arduous process. The method of the present invention, as detailed above, overcomes difficulties encountered with prior art methods and provides a much more rapid and efficient screening procedure to determine whether the mutation occurred at a specific site.

Table II lists eight of the most common mutations in the exons of the CFTR gene. Next to each mutation appears the specific test oligonucleotide to be used to detect this mutation and the labelled dideoxynucleotide which would be incorporated at the 3' end of the specific test oligonucleotide in a normal individual and in an individual having the mutation. Also listed in Table II are three common mutations in the introns of the CFTR gene, the specific test oligonucleotide which may be used to detect and determine the mutation, and the labelled primer extension nucleotide, e.g., dideoxynucleotide which would be incorporated at the 3' end of the test oligonucleotide in normal or mutant individuals.

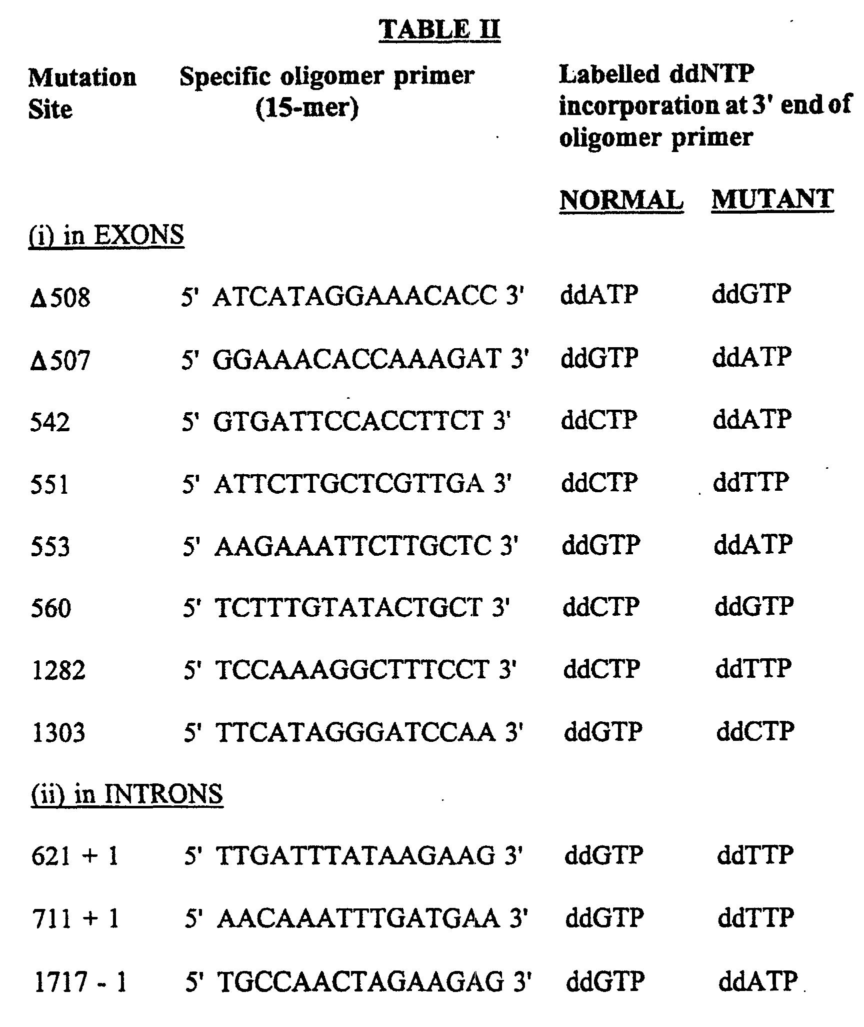

It should be noted that instead of using the specific oligonucleotide primers noted in Table II above, each of which is capable of annealing with the RNA or with only one of the two DNA strands of the CFTR gene at the specific site, it is also possible, when the sample is in the form of DNA, to use an alternative primer specific for the same CFTR gene site but which is complementary to the other DNA strand.

### EXAMPLE 1

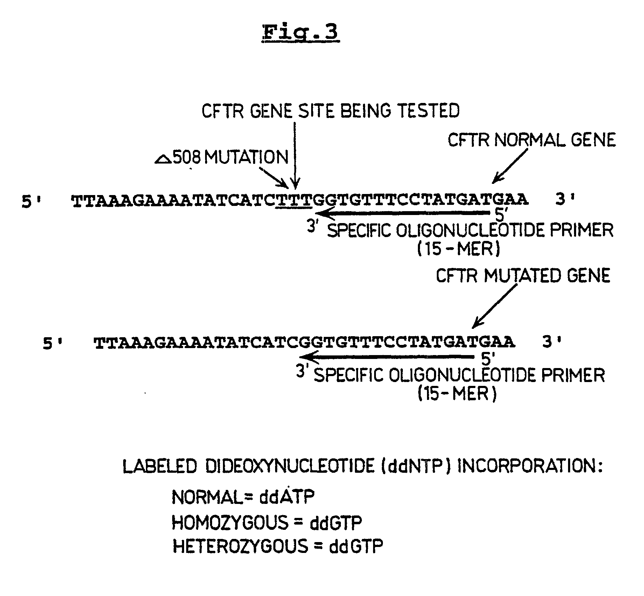

Fig. 3 illustrates the testing of the presence of a Δ508 mutation in a CFTR gene using the basic method. A primer having the sequence 5'ATCATAGGAAACACC3' is annealed to the template DNA and then following an incorporation, or extension, reaction the identity of the incorporated ddNTP in the extended primer is tested in a single vessel. The normal gene contains a T triplet at the tested site and hence the incorporated ddNTP will be a ddATP, and in a mutated gene, where this tripled has been deleted, the incorporated ddNTP will be ddGTP. Incorporation of only ddATP will indicate a normal subject. Incorporation of only ddGTP will indicate a subject which carries two alleles of the mutated gene, i.e., homozygous. Incorporation of both ddATP and ddGTP will indicate that the subject is heterozygous for this mutation.

### EXAMPLE 2

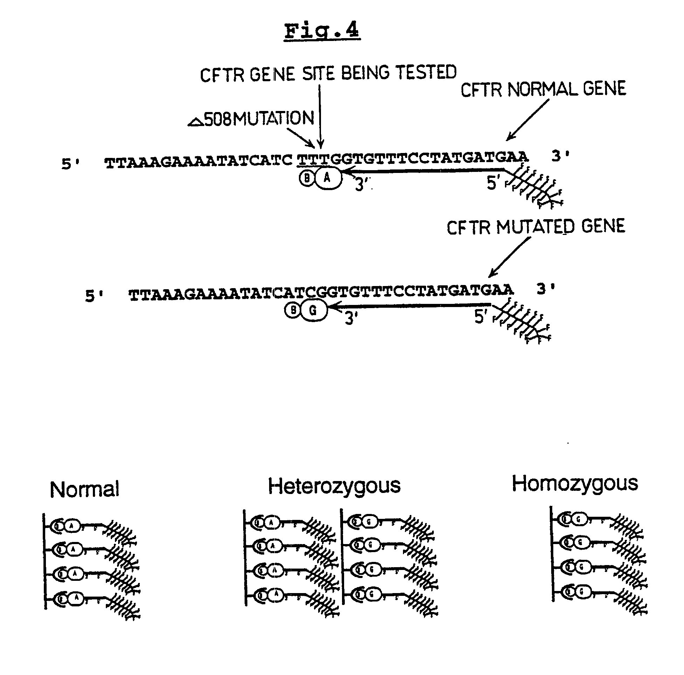

Figure 4 illustrates use of a preferred embodiment of the methods according to the present invention in the testing for the presence of the Δ508 mutation in the CFTR gene. The reaction is performed in two different vessels. One of the vessels contains all the reaction components and biotinylated deoxynucleotide (dATP) or dideoxynuycleotide (ddATP) denoted in the Figure as 'd/ddATP', while the second vessel contains the reaction components but with d/ddGTP. A primer, which may be the same as used in Example 1, but 5' multi-labeled, is annealed to the template DNA. Following the incorporation of the biotinylated nucleotide into the marked primer, the extended and non-extended primers are separated through the binding of the extended primers to streptavidin solid support. Thus, while the extended primer is bound to the streptavidin matrix, the non-extended primer is washed away.

### EXAMPLE 3

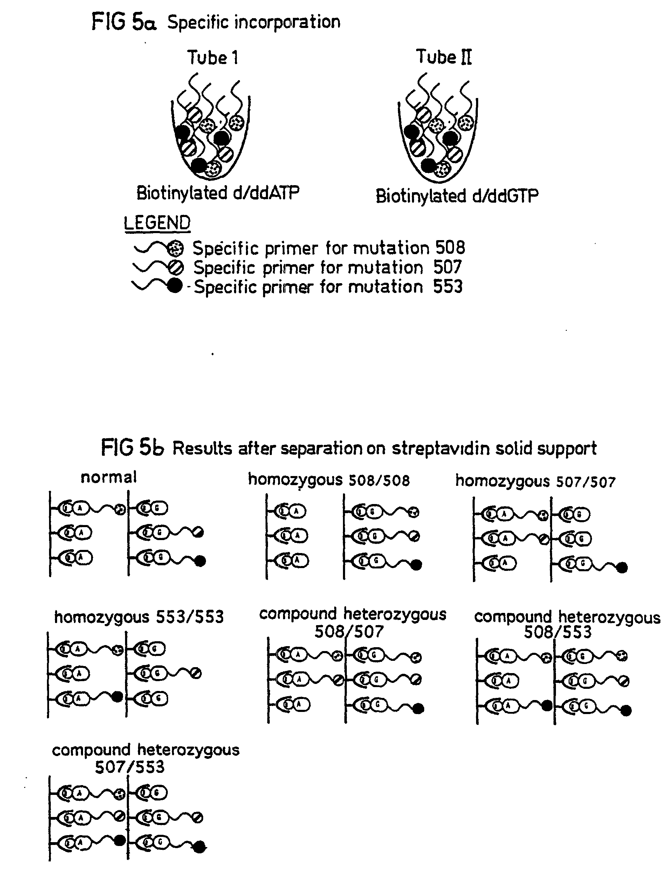

Figure 5 illustrates the simultaneous testing, using a preferred embodiment of the methods according to the present invention, for the presence of three different mutations in the CFTR gene, using two vessels.

One vessel contains all the reaction components and biotinylated d/ddATP while the second vessel includes the reaction components but d/ddGTP.

Three marked primers are used, as follows: 5'ATCATAGGAAACACC3', 5' GGAAACACCAAAGAT3' and 5'AAGAAATTCTTGCTC3'. These are annealed immediately downstream of mutations Δ508, Δ507 and 553, respectively (see also Table II). Proper control of the hybridizing temperature can ensure that no significant cross-hybridization will take place between the primers which could induce false positive or false negative results.

The primer annealed downstream of the Δ508 mutation is 5'-fluorescein multi-labeled (green signal), the primer annealed downstream of the Δ507 mutation is 5'-Texas Red multi-labeled (red signal), while primer annealed downstream of the 553 mutation is 5'-Coumarin multi-labeled (blue signal).

Following the incorporation of the biotinylated nucleotide into the primer, to form an extended primer, the separation between the extended and non-extended primers is performed on streptavidin solid support. While the extended primers are bound to the streptavidin matrix, the non-extended primer is washed away. The results can be analyzed based on the extension or non-extension of the various primers, as shown in Figure 5 and as summarized in Table III.

**TABLE III**

| | | | Results in Two Vessels | | | |
|---|---|---|---|---|---|---|
| Mutation | Normal | Mutated | Color | Normal A/G | Homozygous A/G | Heterozygous A/G |
| Δ508 | A | G | Green | +/- | +/+ | -/+ |
| Δ507 | G | A | Red | -/+ | +/+ | +/- |
| 553 | G | A | Blue | -/+ | +/+ | +/- |

### EXAMPLE 4

### A diagnostic kit for screening or detecting mutations

A diagnostic kit for carrying out a preferred embodiment of the methods according to the present invention detailed above may contain the following constituents:
a) one or more marked oligonucleotide primers, each primer designed to be specific for a particular gene or region in a gene;
b) one or more primer extension units, each including a dideoxynucleotide serving as the extension moiety and further including biotin serving as the separation moiety;
c) suitable buffer in aqueous solution for carrying out the annealing, extension, binding and wash steps of the method;
d) a suitable template-dependent extension enzyme for carrying out the primer extension unit incorporation, or extension, step of the method; and
e) solid support for effecting the separation between extended and non-extended primers.

When the kit is to be used for CFTR gene screening, it may contain any one or all of the specific oligonucleotide primers listed in Table II above for screening or detecting the most common mutations occurring in this gene. When the kit is to be used in the screening for the presence of one or all of the various known genetic diseases, e.g., those listed in Table I above, it may contain any suitable number of the specific oligonucleotide primers for screening for a specific mutation in a particular disease-related gene and in cases where a particular disease-related gene may have one or more mutations, e.g., the CFTR gene, the kit should contain the specific oligonucleotide primers for screening the more common of the mutations, which may be different for different intended populations. When the kit is to be used for blood or tissue typing analysis it may contain any number of the specific oligonucleotide primers, each designed to identify a particular blood or tissue type. Depending on the circumstances, all of the kits may also contain an additional oligonucleotide primer for determining the presence or absence of a DNA sequence corresponding specifically to the presence of a pathogen, for example, the presence of the AIDS virus or a specific strain of such virus, e.g., HIV-I, HIV-II or HIV-III. Accordingly, one kit may be used for testing any number of genes or gene sites within a single gene, and this only requires that the kit contain a number of the specific oligonucleotide primers, all the other components of the kit being the same in all cases.

## Claims

1. Method for determining whether or not one of one or more given nucleotide bases is present at a specific position in a target nucleic acid contained in a sample, said method comprising the steps of:
(a) if the target nucleic acid in said sample is double-stranded, treating said sample to render a region spanning said specific position single-stranded; or, if the target nucleic acid in said sample is single stranded, proceeding directly to step (b); and
(b) forming a hybrid between said target nucleic acid in said sample and a fluorescently labeled oligonucleotide primer, said primer hybridizing to a region on said target that is directly downstream (3') from said specific position, such that a nucleotide base added to the 3'end of said primer would be complementary to said specific position; and
(c) contacting said hybrid with one or more primer extension units, said primer extension unit including an extension moiety and a separation moiety, which is selected from the group consisting of antibodies, antigens, haptens, lectins and carbohydrates, in the presence of a template-dependent extension enzyme under conditions such that if said extension moiety of one of said primer extension units is complementary to said specific position, said primer extension unit will become covalently attached to the 3'end of said primer; and
(d) contacting said primer with a solid support having an affinity for the separation moiety of the primer extension unit, under conditions that promote the attachment of said separation moiety to said suport; and
(e) determining whether said labeled primers have substantially become attached to said solid support, such substantial attachment indicating that the nucleotide base present at said specific position is a base comlementary to one of the one or more extension moieties used, and the substantial absence of such attachment indicating that said nucleotide base is not a base that is complementary to one of the one or more extension moieties used.

2. The method according to claim 1, wherein said fluorescently labeled primer has multiple label moieties attached thereto.

3. The method according to claim 1 or claim 2, wherein said fluorescently labeled primer has multiple label moieties attached at multiple locations on said primer.

4. The method according to any of the preceding claims, wherein the separation moiety of step (c) is biotin.

5. The method according to claim 4, wherein said solid support of step (d) is an avidin-coated microtiter plate well.

6. The method according to any of the preceding claims, wherein unreacted primer extension units are not removed from the sample resulting from step (c) prior to commencing step (d).

7. The method according to any of the preceding claims wherein said method determines whether or not given bases are present in any of a plurality of specific positions in one or more target nucleic acids contained in a sample, wherein said method is modified in that hybrids are formed in said step (b) between said target nucleic acids in said sample and a plurality of said labeled oligonucleotide primers, one for each of said plurality of specific positions

## Patentansprüche

1. Verfahren zur Bestimmung, ob eine oder mehrere gegebene Nukleotidbasen an einer spezifischen Position in einer in einer Probe enthaltenen Target-Nukleinsäure enthalten sind oder nicht, das folgende Schritte umfaßt:
(a) wenn die Target-Nukleinsäure in der Probe doppelsträngig ist, wird die Probe derart behandelt, daß ein Bereich, der die spezifische Position überspannt, einsträngig wird; oder, wenn die Target-Nukleinsäure in der Probe einsträngig ist, geht man direkt zum Schritt (b); und
(b) es wird ein Hybrid zwischen der Target-Nukleinsäure in der Probe und einem fluoreszierend markierten Oligonukleotid-Primer gebildet, wobei der Primer zu einem Bereich an dem Target hybridisiert, der direkt unterhalb (3') der spezifischen Position liegt, so daß eine Nukleotidbase, die an das 3'-Ende des Primers angefügt ist, zu der spezifischen Position komplementär wäre; und
(c) das Hybrid wird mit einer oder mehreren Primer-verlängerungseinheiten in Kontakt gebracht, wobei die Primer-Verlängerungseinheit einen Verlängerungsteil und einen Trennungsteil umfaßt, der aus der Gruppe von Antikörpern, Antigenen, Haptenen, Lectinen und Kohlehydraten ausgewählt ist, in Gegenwart eines templatabhängigen Verlängerungsenzyms unter solchen Bedingungen, daß dann, wenn der Verlängerungsteil einer der Primer-Verlängerungseinheiten zu der spezifischen Position komplementär ist, die Primen-Verlängerungseinheit kovalent an das 3'-Ende des Primers angelagert wird; und
(d) der Primer wird mit einem festen Träger mit einer Affinität für den Trennungsteil der Primer-Verlängerungseinheit unter Bedingungen in Kontakt gebracht, die die Anlagerung des Trennungsteils an den Träger fördern; und
(e) es wird bestimmt, ob die markierten Primer im wesentlichen an den festen Träger angelagert sind, wobei eine solche wesentliche Anlagerung angibt, daß die an der spezifischen Position vorliegende Nukleotidbase eine zu einem oder mehreren verwendeten Verlängerungsteilen komplementäre Base ist, und wobei die wesentliche Abwesenheit einer solchen Anlagerung angibt, daß die Nukleotidbase keine Base ist, die zu einem oder mehreren verwendeten Verlängerungstielen komplementär ist.

2. Verfahren nach Anspruch 1, bei welchem an den fluoreszierend markierten Primer mehrere Markierungsteile angelagert sind.

3. Verfahren nach Anspruch 1 oder Anspruch 2, bei welchem der fluoreszierend markierte Primer mehrere Markierungsteile hat, die an mehreren Orten an dem Primer angelagert sind.

4. Verfahren nach einem der vorhergehenden Ansprüche, bei welchem der Trennteil von Schritt (c) Biotin ist.

5. Verfahren nach Anspruch 4, bei welchem der feste Träger von Schritt (d) eine Avidin-beschichtete Mikrotiterplattenmulde ist.

6. Verfahren nach einem der vorhergehenden Ansprüche, bei welchem unreagierte Primer-Verlängerungseinheiten nicht aus der sich aus dem Schritt (c) ergebenden Probe entfernt werden, ehe mit dem Schritt (d) begonnen wird.

7. Verfahren nach einem der vorhergehenden Ansprüche, bei welchem das Verfahren bestimmt, ob oder nicht gegebene Basen in einer aus einer Vielzahl von spezifischen Positionen in einer oder mehreren Target-Nukleinsäuren anwesend sind, die in einer Probe enthalten sind, wobei das Verfahren dadurch modifiziert ist, daß Hybride bei dem Schritt (b) zwischen den Target-Nukleinsäuren in der Probe und einer Vielzahl der markierten Oligonukleotid-Primer gebildet werden, einem für jede aus der Vielzahl spezifischer Positionen.

## Revendications

1. Procédé pour déterminer si ou non une ou plusieurs bases de nucléotide est présente à une position spécifique dans un acide nucléique cible contenu dans un spécimen, ledit procédé comprenant les étapes suivantes:
(a) si l'acide nucléique cible dans ledit spécimen est à deux brins, traiter ledit spécimen pour rendre une région enjambant ladite position spécifique à un brin; ou, si l'acide nucléique cible dans ledit spécimen est à un brin, procéder directement à l'étape (b); et
(b) former un hybride entre l'acide nucléique cible dans ledit spécimen et une amorce d'oligonucléotide marquée fluorescemment, ladite amorce hybridant à une région sur ladite cible qui se trouve directement an aval (3') de ladite position spécifique, de manière qu'une base nucléotide ajoutée à l'extrémité 3' de ladite amorce serait complémentaire à ladite position spécifique; et
(c) contacter ledit hybride avec une ou plusieurs unités d'allongement d'amorce, ladite unité d'allongement d'amorce incluant un composant d'allongement et un composant de séparation qui est choisi parmi le groupe consistant d'anticorps, antigènes, haptènes, lectines et d'hydrates de carbone, en présence d'une enzyme d'allongement dépendant de la matrice, dans de telle conditions que, si ledit composant d'allongement de l'une des unités d'allongement d'amorce est complémentaire à ladite position spécifique, ladite unité d'allongement d'amorce sera attachée à l'extrémité 3' de ladite amorce d'une manière covalente; et
(d) contacter ladite amorce avec un support solide ayant une affinité pour le composant de séparation de l'unité d'allongement d'amorce, dans des conditions qui favorisent l'attachement dudit composant de séparation audit support; et
(e) déterminer si lesdites amorces marquées se sont essentiellement attachées audit support solide, un tel attachement essentiel indiquant que la base de nucléotide présente à ladite position spécifique est une base complémentaire à l'une parmi ledit ou plusieurs composants d'allongement utilisés, et l'absence essentielle d'un tel attachement indiquant que ladite base de nucléotide n'est pas une base qui est complémentaire à l'un ou plusieurs des composants d'allongement utilisés.

2. Procédé selon la revendication 1, dans lequel plusieurs composants de marquage sont attachés à ladite amorce marquée fluorescemment.

3. Procédé selon la revendication 1 ou la revendication 2, dans lequel plusieurs composants de marquage sont attachés, à plusieurs sites sur ladite amorce, à ladite amorce marquée fluorescemment.

4. Procédé selon l'une quelconque des revendications précédentes, dans lequel le composant de séparation de l'étape (c) est la biotine.

5. Procédé selon la revendication 4, dans lequel le support solide de l'étape (d) est un creux d'une plaque de microtiter enduite d'avidine.

6. Procédé selon l'une quelconque des revendications précédentes, dans lequel les unités d'allongement d'amorce non-réagies ne sont pas éliminées du spécimen résultant de l'étape (c) avant que l'étape (d) ne soit commencée.

7. Procédé selon l'une quelconque des revendications précédentes, dans lequel ledit procédé détermine si ou non des base données sont présentes dans l'une quelconque d'une multiplicité de positions spécifiques dans un ou plusieurs acides nucléiques, ledit procédé étant modifié en ce que des hybrides sont formés à l'étape (b) entre lesdits acides nucléiques cible dans ledit spécimen et une multiplicité desdites amorces d'oligonucléotide marquées, un pour chacune parmi la multiplicité de positions spécifiques.
